Europäisches Patentamt

European Patent Office

Office européen des brevets

(19)

(11) Publication number: 0 200 113
A2

(12) # EUROPEAN PATENT APPLICATION

(21) Application number: 86105375.9

(51) Int. Cl.⁴: **C 12 Q 1/68**

(22) Date of filing: 18.04.86

(30) Priority: 30.04.85 US 729001

(43) Date of publication of application:
05.11.86 Bulletin 86/45

(84) Designated Contracting States:
AT BE CH DE FR GB IT LI LU NL SE

(71) Applicant: Pandex Laboratories, Inc.
909 Orchard Street
Mundelein Illinios 60062(US)

(72) Inventor: Jolley, Michael E.
34469 North Circle Drive
Round Lake Illinois 60073(US)

(72) Inventor: Ekenberg, Steven J.
404 Pleasant View
McHenry Illinois 60050(US)

(74) Representative: Strehl, Schübel-Hopf, Groening, Schulz
Widenmayerstrasse 17 Postfach 22 03 45
D-8000 München 22(DE)

(54) A method of solid phase nucleic acid hybridization assay incorporating a luminescent label.

(57) A method of solid phase nucleic acid hybridization assay which incorporates a first fragment of nucleic acid bound to a solid carrier comprising water insoluble particles and a second fragment of nucleic acid bound to a luminescent label. Hybridization occurs while the solid carrier is suspended and the luminescence is measured after the solid carrier is concentrated by microfiltration.

EP 0 200 113 A2

Pandex Laboratories, Inc.
EPA-27 643

86105395.9

0200113

A METHOD OF SOLID PHASE NUCLEIC ACID
HYBRIDIZATION ASSAY INCORPORATING A
LUMINESCENT LABEL

## Background of the Invention

### 1. Field of the Invention

This invention relates to a method of solid phase nucleic acid hybridization assay incorporating a luminescent label for the detection and/or quantitation of nucleic acid from a variety of biological sources.

### 2. Description of the Prior Art

Nucleic acid hybridization is a technique which has been used for the detection of single-stranded polynucleotides from a variety of biological sources. The technique has been used for the detection of microorganisms, such as bacteria and viruses, mammalian genes (for example: sickle cell hemoglobin), oncogenes, and a variety of other genes of medical importance.

In the most common nucleic acid hybridization assay, the sample nucleic acid (usually present as double-stranded DNA or RNA) is first converted to single-stranded form and immobilized onto a solid carrier. It may or may not be cut by restriction nucleases beforehand. Usually nitrocellulose paper is employed as the solid carrier, but other papers have been used successfully. The sample nucleic acid may also be electrophoresed into a gel matrix. The immobilized sample nucleic acid is then hybridized with a labeled nucleic acid probe which is complementary to specific sequences on the sample nucleic acid. The probe nucleic acid may be labeled with radioactive, enzyme, fluorescent, chemiluminescent or bioluminescent molecules, which are detected in a subsequent step.

A number of procedures are known for binding sample nucleic acid to a solid carrier, including: (1) binding single stranded DNA to nitrocellulose (E. Southern, J. Mol. Biol, Vol. 98, pp. 503-527 (1975); incorporated herein in its entirety by reference thereto, all following citations to articles, patents, technical bulletins or other publications similarly incorporated herein by reference thereto); (2) binding DNA to diazobenzyloxymethyl-paper (G. Wahl, et al., Proc. Nat. Acad. Sci., Vol. 76, pp. 3683-387 (1979)); and (3) binding DNA covalently to cellulose (B. Noles, et al., Cell, Vol. 5, pp. 301-310 (1975)). There are many methods available to hybridize probes in solution to DNA or RNA immobilized on a filter. These methods differ in the solvent and the temperatures used, the labeled probe and its specific activity, and the use of compounds such as dextran sulfate that increase the rate of association.

Detection of hybridized molecules was traditionally accomplished by labeling DNA to high specific activity by a nick translation reaction using deoxnucleotide alpha $^{32}$P triphosphates (P. Rigby, et al., Mol. Biol., Vol. 133, pp. 237-251 (1977)) and hybridizing this DNA to the sample DNA immobilized on the filter. In a move to eliminate radioactivity, a biotin molecule was incorporated through the C5 residue of dUTP (P. Langer, et al., Proc. Nat. Acad. Sci., Vol. 78 (22), pp. 6633-6637 (1981). By incorporating this biotinylated dUTP into DNA by a nick translation or terminal transferase procedure (cf. "The Enzo Bio-Probe System: Terminal Labeling Kit," Enzo Biochem, Inc., New York, New York 10013), hybridized DNA could be detected by

- 3 -

0200113

using immunological techniques for the detection of biotin or by using the high affinity of avidin (conjugated to an enzyme or fluorescent label) to biotin incorporated in the DNA hybrids. In variations of the hybridization assay, sandwich hybridizations are described as utilizing two probes: probe #1 was immobilized on a membrane to capture the sample DNA and probe #2 was a radioactive labeled strand of DNA to act as the signal probe. If no sample was present, unbound labeled DNA was washed away giving rise to the absence of signal, except for background noise. (M. Ranki, Gene, Vol. 21, pp. 77-85 (1983); U.S. Patent No. 41486,539 issued to Ranki, et al., on December 4, 1984).

All of the above prior art hybridization assays are slow, cumbersome, time consuming and prone to procedural and subjective errors. The need exists for a fast, reliable, highly sensitive method for nucleic acid hybridization.

SUMMARY OF THE INVENTION

In accordance with the invention, an improved method of nucleic acid hybridization assay is provided for the detection and/or quantitation of nucleic acids, including DNA and RNA, in a liquid sample. The assay may be directed at any nucleic acid without regard to its source provided that the nucleic acid can be rendered single-stranded and available for hybridization. Rendering such nucleic acid single-stranded and available for hybridization will generally provide the liquid sample which will be assayed.

The present hybridization assay can in principle be used for detection and/or quantitation of DNA or RNA containing organisms, such as viruses, bacteria, fungi and yeast. The present hybridization assay can be used, for example, in medical microbiology, veterinary microbiology, food hygiene investigations, and microbial diagnostics of plant diseases. Suitable source materials are animal and plant tissue homogenates, blood, feces and nasal and urethral mucous. It is believed that the present hybridization assay is sufficiently sensitive to detect microbe levels present in clinical samples. Preliminary enrichment of the microbe present in a sample by cultivation is possible before the assay is performed.

The present hybridization assay can be used, for example, to detect nucleic acids present in (1) bacterial respiratory infections such as beta-hemolytic streptococci, Hemophilus influenzae, pneumococci, Mycoplasma pneumoniae, and mycobacteria; (2) viral respiratory infections such as influenza A, influenza B, parainfluenza (types 1, 2 and 3), respiratory syncytial virus, adenoviruses, coronaviruses and rhinoviruses; (3) bacterial diarrhoeas such as salmonellae, shigellae, Yersinia enterocolitica, enterotoxigenic, E. coli, Clostridium difficile and campylobacter; (4) viral diarrhoeas such as rotaviruses, parvoviruses, adenoviruses, and enteroviruses; (5) bacterial venereal diseases such as Neisseria gonorrhoeae, Treponema pallidum, Chlamydia trichomatis; (6) viral venereal diseases such as Herpes simplex virus; (7) yeast venereal diseases such as Candida albicans, protozoa venereal diseases such as Trichomonas vaginalis; (8) bacterial sepsis such as beta-hemolytic streptococci (group A), pneumococci, enterobacteria; and (9) bacterial food hygiene such as salmonellae, and Clostridium perfringens.

In accordance with the present hybridization assay, source nucleic acids are rendered single-stranded and available for hybridization, thereby forming a liquid sample of measurable volume containing single-stranded sample nucleic acids to be assayed. A single-stranded first fragment of nucleic acid, having a nucleotide sequence of at least 15 bases, is attached to a solid carrier. The solid carrier comprises a plurality of water insoluble particles of about 50 microns or less in diameter. A single-stranded second fragment of nucleic acid, having a nucleotide sequence of at least 15 bases, is attached to a luminescent label or a luminescent label acceptor to which a luminescent label may be later attached. As discussed below, the complementary specificity of the first and second fragments of nucleic acid may be determined according to desired choice of assay configuration. Following hybridization involving the sample nucleic acid, first fragment of nucleic acid and second fragment of nucleic acid, the solid phase, constituting the solid carrier and components attached thereto, is separated from the liquid phase. If the hybridizations are performed sequentially rather than simultaneously, sequential separating of solid phase from liquid phase may be required. The luminescence of the separated solid phase will allow detection and/or quantitation of sample nucleic acid in the liquid sample according to the chosen assay configuration.

The present hybridization assay is an improvement over existing assay configurations because (1) the solid phase comprises the first fragment of nucleic acid attached to microparticles and (2) at least one of the hybridization reactions between the solid phase and the liquid phase containing either sample nucleic acid or second fragment of nucleic acid or both occurs while the solid phase is in a substantially suspended state. It is also an improvement because at the completion of the hybridization reactions, the solid phase is concentrated by microfiltration to a volume substantially less than the volume of the liquid sample. By proper choice of the size of the concentrate, the luminescence of substantially all of the luminescent label attached to the concentrated solid carrier may be measured.

## PREFERRED AND ALTERNATIVE EMBODIMENTS

The hybridization assay utilizes a solid carrier comprising a plurality of water insoluble particles of about 50 microns or less in diameter. For example, the particles may be polymeric particles such as polystyrene latex. The particles may have a preferred diameter in the range of about 0.2 microns to about 50 microns.

The particles may be substantially transparent to the luminescence resulting from excitation of the fluorescent label, phosphorescent label, atomic fluorescent label, chemiluminescent label, or bioluminescent label. The particles may also be substantially transparent to a beam exciting the label in the case of fluorescent label, phosphorescent label, or atomic fluorescent label. The luminescent label may have an optical luminescence which is measured by a device having an excitation beam with a cross-section size within the range of about 1 to 5 mm. The luminescence may be measured after the solid carrier has been concentrated by microfiltration to have a cross-section size within the range of about 1 to 5 mm.

The second fragment of nucleic acid may be attached to a luminescent label acceptor to which a luminescent label may be attached after completion of some or all of the hybridization reactions. For example, the second fragment of nucleic acid may be attached to a luminescent label acceptor by reaction of biotin with the second fragment. Luminescent label will become attached to the second fragment by reaction of a avidin-luminescent label conjugate with the luminescent label acceptor attached to the second fragment.

The speed and sensitivity of the hybridization assay are enhanced by performing the hybridization reactions while the solid carrier is substantially suspended. The large surface area of the solid carrier can bring significant quantities of the second fragment of nucleic acid, and alternatively sample nucleic acid or first fragment of nucleic acid attached thereto, into the reaction. Substantially suspending the solid carrier distributes the hybridization reactants attached thereto throughout the liquid medium in which the hybridization reactions occur. This enhances rapid and complete hybridization reactions.

The solid carrier may be concentrated to a volume substantially less than the volume of the liquid sample by microfiltration. This yields a two fold advantage. First, the sample nucleic acid, or in the case of some assay configurations the luminescent label or luminescent label acceptor which correlates directly or inversely to the sample nucleic acid, may be concentrated prior to detection and/or quantitation, thereby increasing the sensitivity of the assay by a factor substantially identical to the concentration factor. Second, the volume of the solid carrier may be concentrated to a volume where a luminescent device such as, for example, a fluorometer, may observe substantially all of the luminescent label, thereby increasing the sensitivity of the assay by a factor substantially identical to the ratio of the amount of luminescent label contained in the concentrated volume to the amount of label which would be excited by the beam if the liquid containing the solid carrier were not concentrated.

A typical solid carrier, for example, might comprise a 0.3 percent by volume suspension of 0.8 μm particles to which the first fragment of nucleic acid is attached. The solid carrier contained in 20 μl of this suspension has a surface area of 4.4 $cm^2$ and a volume of 0.06 μl. In a sandwich configuration, the sample nucleic acid contained in 100 ul of liquid sample will be concentrated 1,667-fold after reaction with the first fragment of nucleic acid and microfiltration. The small volume of solid carrier can be conveniently filtered to form a 1-5 mm in size (i.e., diameter). The sample nucleic acid, attached to the solid carrier by hybridization with the first fragment, may be reacted with the labeled second fragment of nucleic acid. This will allow the fluorescence of substantially the whole solid phase to be observed by a front face fluorometer. Without such concentration, a standard fluorometer would have excited only 1-2 percent of the label attached to the suspended solid carrier. Thus by concentration, the 1,667-fold increase in sensitivity due to concentration is compounded by the about 70-fold increase in sensitivity due to measuring substantially all of the luminescent label yields a total increase in sensitivity of in excess of 100,000 X.

In carrying out the present hybridization assay, a number of alternative assay configurations are available. The following illustrates some of these possible assay configurations.

In a sandwich assay configuration, solid carrier bound three-component hybrid molecules are allowed to form by complementary base pairing of sample nucleic acid contained in a liquid sample of measurable volume, first fragment of nucleic acid having a nucleotide sequence of at least 15 bases and attached to said solid carrier, and second fragment of nucleic acid having a nucleotide sequence of at least 15 bases and attached to said luminescence label or said label acceptor. The first fragment is capable of hybridizing with the sample nucleic acid and the second fragment is capable of hybridizing with the sample nucleic acid but not capable of hybridizing with the first fragment. The resulting hybrid sandwich molecule attached to the solid carrier is designated herein as "three-component" because in addition to the label or label acceptor it contains the sample nucleic acid, the first fragment and the second fragment of nucleic acid.

The solid carrier bound three-component hybrid molecules to which luminescent label or label acceptor is attached is separated from luminescent label or label acceptor not attached to said three-component hybrid molecules. Complementary base pairing between the sample nucleic acid and the first fragment of nucleic acid occurs while the solid carrier, which comprises a plurality of water insoluble particles of about 50 microns or less in diameter and to which the first fragment is attached, is in a substantially suspended state. The luminescence of the solid carrier bound three-component hybrid molecules is measured after the solid carrier has been concentrated by microfiltration to a volume substantially less than the volume of the liquid sample.

As an embodiment of the sandwich configuration, the three-component hybrid molecules may be formed by simultaneous complementary base pairing of the sample nucleic acid, the first fragment and second fragment of nucleic acid while the solid carrier, to which the first fragment is attached, is in a substantially suspended state. According to this embodiment, the first fragment and second fragment are added to the liquid sample so that hybridization between the first fragment and sample nucleic acid and hybridization between sample nucleic acid and second fragment occur substantially simultaneously. After formation of the three-component hybrid molecules, the solid carrier may be drawn down by microfiltration.

In the case where the label is already attached to the three-component hybrid molecules, the luminescence thereof may be measured. In the case where a luminescent label acceptor is attached to the three-component hybrid molecules, luminescent label may be attached to the acceptor while the solid carrier is concentrated or the solid carrier may be resuspended for such addition of luminescent label. In the latter case, the solid carrier may again be drawn down by microfiltration prior to measurement of the luminescent label.

As an alternative sandwich embodiment, the three-component hybrid molecules may be formed by first allowing hybridization of the first fragment of nucleic acid with the sample nucleic acid while the solid carrier, to which the first fragment is attached, is in a substantially suspended state. This first hybridization reaction will result in two-component hybrid molecules containing the first fragment and the sample nucleic acid. Second, the three-component hybrid molecules are allowed to form by complementary base pairing of the second fragment of nucleic acid with the sample nucleic acid contained in the two-component hybrid molecules.

The solid carrier may or may not be drawn down by microfiltration after the first hybridization reaction which produces the two-component molecule. The second hybridization reaction may occur while the solid carrier is in a concentrated state or after the solid carrier has been resuspended. In any case, the luminescent label would be measured while concentrated in accordance with the procedures described in the foregoing sandwich embodiment.

As a still further sandwich alternative, the sample nucleic acid may be reacted with the second fragment of nucleic acid prior to addition of and reaction with the first fragment of nucleic acid attached to the solid carrier.

In sequential saturation, competitive displacement, competitive binding, and related assay configurations, two distinct species of solid carrier bound two-component hybrid molecules are allowed to form by complementary base pairing of (1) sample nucleic acid to the assayed contained in a liquid sample of measurable volume, (2) first fragment of nucleic acid having a nucleotide sequence of at least 15 bases and which is attached to the solid carrier and (3) second fragment of nucleic acid having a nucleotide sequence of at least 15 bases and to which is attached a luminescent label or a luminescent label acceptor. The first fragment is capable of hybridizing with the sample nucleic acid. The second fragment of nucleic acid is capable of hybridizing with the first fragment but not capable of hybridizing with the sample nucleic acid. The resulting two species of hybrid molecules attached to the solid carrier is designated herein as "two-component" because one species contains the first fragment and the sample nucleic acid while the other species contains the first fragment and the second fragment of nucleic acid.

The two species of solid carrier bound two-component hybrid molecules to which luminescent label or label acceptor is attached is separated from luminescent label or label acceptor not attached to said two species of solid carrier bound two-component hybrid molecules. Complementary base pairing between (1) the first fragment of nucleic acid and (2)(i) the sample nucleic acid or (ii) the second fragment of nucleic acid occurs while the solid carrier, which comprises a plurality of water insoluble particles of about 50 microns or less in diameter and to which the first fragment is attached, is in a substantially suspended state. The luminescence of the solid carrier bound two-component hybrid molecules is measured after the solid carrier has been concentrated by microfiltration to a volume substantially less than the volume of the liquid sample.

As an embodiment of the sequential saturation configuration, the two species of solid carrier bound two-component hybrid molecules may be formed by first allowing hybridization of the sample nucleic acid with an excess of first fragment of nucleic acid while the solid carrier to which the first fragment of nucleic acid is attached is in a substantially suspended state, and second, allowing hybridization of (1) first fragment of nucleic acid which was not hybridized by sample nucleic acid with (2) second fragment of nucleic acid. According to this embodiment, there are an excess number of first fragment sites on the solid carrier with the result that the sample nucleic acid can hybridize with only a portion of the available first fragment sites. In the second hybridization reaction the second fragment of nucleic acid to which the label or label acceptor is attached will fill the remaining sites. In this assay configuration, the amount of label in the solid phase will vary inversely with the amount of sample nucleic acid present in the liquid sample.

As an embodiment of the competitive displacement configuration, the two species of solid carrier bound two-component hybrid molecules are allowed to form by first allowing hybridization of the first fragment of nucleic acid with the second fragment of nucleic acid while the solid carrier to which the first fragment of nucleic acid is attached is in a substantially suspended state, and second, allowing displacement of the hybridized second fragment of nucleic acid by hybridization of the first fragment of nucleic acid with the sample nucleic acid. In this assay configuration, the second fragment hybridizes with substantially all of available first fragment sites. Under suitable reaction conditions, hybridized first fragment will be displaced by sample nucleic acid until the competiting hybridization reactions reach equilibrium. In the case of this assay configuration, the luminescence of the solid phase will vary inversely with the amount of sample nucleic acid in the liquid sample.

As an embodiment of the competitive binding configuration, the two species of solid carrier bound two-component molecules are allowed to form by simultaneous complementary base pairing of (1) sample nucleic acid and (2) second fragment of nucleic acid in competition for first fragment of nucleic acid, while the solid carrier to which the first fragment of nucleic acid is attached, is in a substantially suspended state.

As an alternative embodiment of the competitive binding configuration, the two species of solid carrier bound two-component hybrid molecules are allowed to form by first allowing hybridization of sample nucleic acid with the first fragment of nucleic acid while the solid carrier, to which the first fragment of nucleic acid is attached, is substantially suspended, and second, allowing competition for such first fragment of nucleic acid by hybridization of second fragment of nucleic acid with such first fragment of nucleic acid. In both of the above competitive configurations, sample nucleic acid and the second fragment of nucleic acid compete for the first fragment of nucleic acid. Such competitive hybridization reactions are generally run to equilibrium, but sensitivity of competitive binding assays is often enhanced by first reacting sample nucleic acid with the first fragment of nucleic acid.

In yet another competitive binding configuration, two species of two-component hybrid molecules, one species bound to the solid carrier and the other species not bound, are allowed to form by comlementary base pairing of (1) sample nucleic acid to be assayed contained in a liquid sample of measurable volume, (2) first fragment of nucleic acid having a nucleotide sequence of at least 15 bases and which is attached to a solid carrier, and (3) second fragment of nucleic acid having a nucleotide sequence of at least 15 bases and to which is attached a luminescent label or a label acceptor to which a luminescent label may be attached subsequent to the formation of two species of two-component hybrid molecules. First fragment of nucleic acid is not capable of hybridizing with the sample nucleic acid, while the second fragment of nucleic acid is capable of hybridizing with sample nucleic acid or the first fragment of nucleic acid.

The species of bound two-component hybrid molecules are separated from the species of unbound two-component hybrid molecules. Complementary base pairing between (1) the first fragment of nucleic acid and (2) the second fragment of nucleic acid occurs while the solid carrier comprising a plurality of water insoluble particles of about 50 microns or less in diameter, to which the first fragment of nucleic acid is attached, is in a substantially suspended state. The presence of the luminescent label attached to solid carrier bound two-component hybrid molecules is measured while the solid carrier has been concentrated by microfiltration to a volume substantially less than the volume of the liquid sample.

As an embodiment of the second type of competitive binding configuration, the two species of two-component hybrid molecules are allowed to form by simultaneous complementary base pairing of (1) sample nucleic acid and (2) first fragment of nucleic acid in competition for second fragment of nucleic acid, while the solid carrier, to which the first fragment of nucleic acid is attached, is in a substantially suspended state.

All of the above assay configurations may be used to detect and/or quantitate nucleic acid. The presence of nucleic acid in a sample will be indicated by the presence or absence of signal according to the assay configuration chosen. Background noise can be accounted for in determining whether a signal is present or absent by performing assays on standards in which no sample nucleic acid is present. Nucleic acid in a sample may be quantitated by known methods where the signal attributable to the presence of sample nucleic acid is correlated to the amount of signal for known standards containing varying amounts of such species of sample nucleic acid or nucleic acid which is cross-reactive with the sample.

The following examples will show the applicability of the present hybrid assay to the detection of calf thymus DNA. The examples given are characterized as sandwich assays; however, these examples serve only to demonstrate the versitility and sensitivity of the present hybrid assay and are not meant to limit its use in any way.

### EXAMPLE 1

Calf thymus DNA was mercurated by a modification of the procedure described by R. Dale, et al., Biochem., Vol. 14(11), pp. 2447-2457 (1975). Calf thymus DNA was added to 2 ml of 0.1 M sodium acetate buffer pH 6.0 at a concentration of $7 \times 10^{-4}$M (146 nmole/OD unit) and stirred on a magnetic stirrer overnight to facilitate solubility. These solutions were heated to 50°C at which time a fifteen-fold molar excess of mercuric acetate was added. The sample was removed at 60 minutes and placed on ice. To this solution lithium chloride was added to a concentration of 0.009 M. The samples were extracted six times with equal volumes of ethyl acetate to remove the excess mercuric chloride. The mercurated DNA was ethanol precipitated, washed with cold absolute ethanol and resuspended in medium salt restriction endonuclease buffer (50 mM NaCl, 10mM Tris pH 7.4, and 10 mM MgSO4). The DNA was digested with the following restriction enzymes: BgL II, Hind II and Hind III using 1 unit/$\mu$g of DNA (unit defined by and material available commercially from Ammersham, Inc., Arlington Heights, Illinois) until a 500 base pair fragment was detected on a 0.7% agarose gel. The DNA was digested as described above, run on a gel, extracted by electroelution into troughs, ethanol precipitated and resuspended in 0.1 M sodium acetate buffer pH 6.0 at a concentration of 500 $\mu$g/ml. This DNA was prepared for application to the sulfhydryl particles and to act as the first fragment of nucleic acid.

### EXAMPLE 2

To a 5 ml suspension of 5% w/v amino particles (0.63 u dia., cat. # 30-030-1 (Pandex Laboratories, Inc., Mundelein, Illinois), 5 mls of 0.1 M phosphate buffer pH 7.0 and a solution containing 12.5 mg (3-(2-pyridyldithio) propionic acid N-hydroxy succinimide ester) (SPDP) (according to J. Carlson, et al., Biochem J., Vol. 173, pp. 723-737 (1978)) in 0.5 ml of dimethyl sulfoxide was added. The mixture was stirred for one hour at room temperature and then centrifuged at 6000 rpm for thirty minutes. The particles were washed with an equal volume of phosphate buffer and centrifuged as before. The resulting 2-pyridyl disulfide modified particles were resuspended in 5 mls of

0200113

0.1 M acetate buffer pH 5.0. To this solution 40 mg of 1,4-ditihiothreitol was added and stirred for thirty minutes at room temperature. The particles were centrifuged as before. The supernatant was decanted and filtered through a piece of 0.6 u Whatman filter paper. The thiol group content of the particles was estimated by measuring the absorbance of the supernatant at 343 nm for released pyridine-2-thione using a molar extinction coefficient of $8.08 \times 10^{-3}$ M-1 cm-1 (T. Stuchbury, et al., Biochem., Vol. 151, pp. 417-   (1975)). The absorbance of the supernatant was found to be 4.2 O.D. units which is equivalent to $10.5 \times 10^{-6}$ moles of thiol groups per gram of solids. The particles were washed one time with deionized water, centrifuged as before and resuspended in 5 ml of 0.1 M phosphate buffer pH 7.5.

                    EXAMPLE 3

        1 ml of 0.5% w/v sulfhydryl particles were centrifuged at 6000 rpm for 20 minutes. The supernatant was decanted and the particles stored on ice. The double stranded mercurated calf thymus DNA was heated for five minutes at 100°C for denaturation and rapidly chilled on ice. After ten minutes, the resulting single stranded DNA solutions were added to the particles and incubated in the cold overnight. The following day the particles were centrifuged, supernatant decanted and washed with 0.1 M NaCl to remove excess unbound DNA. The resulting particles, to which was bound first fragment of nucleic acid, were resuspended to 0.25% w/v in 0.1M phosphate buffer pH 7.0.

                    EXAMPLE 4

        Calf thymus DNA was digested as described before to yield a band on an agarose gel approximately 300 base pairs long.  15 micrograms were excised from a gel and used for the tailing reaction of DNA with biotin-11-dUTP.  Tailing of the DNA was accomplished by known procedures ("The Enzo Bio-Probe System - Terminal Labeling Kit," Enzo Biochem, Inc., New York, New York 10013).  The DNA was digested by Pst I using 1 unit/µg of DNA (unit defined by and material available from Ammersham, Inc., Arlington Heights, Illinois) to give a fragment size of 300 base pairs as observed by agarouse gel electrophoresis.  The terminal tailing reaction was allowed to run for ninety minutes to achieve a total nucleotide incorporation of 7.5 nmoles.  The estimated content of biotinylated dUTP was 3.75 nmoles per microgram of DNA.

## EXAMPLE 5

Streptavidin-B-phycoerythrin (SAV-BPE) was synthesized according to known procedures ("Notes on Constructing Phycobiliproteins," Applied Biosystems, Inc., Foster City, California 94404; "Conjugations of Phycobiliproteins to Biological Molecules," Molecular Probes, Inc., Junction City, Oregon 97448).  16 µl of (3-(2-pyridyldithio)propionic acid N-hydroxy succinimide ester) (SPDP, 1.3 mg/ml mixed fresh in anhydrous methanol) was added to 1.45 mg of SAV and incubated for forty minutes at room temperature.  37.5 ul of dithiothreitol (1M in coupling buffer, 0.1 M sodium phosphate, 0.1 M sodium chloride pH 7.4) was added to 5 mg of SPDP derivatized BPE and incubated at room temperature.  These two mixtures were dialyzed together against two changes of coupling buffer (4 liters each) in the dark at 4°C.  After dialysis the two solutions were combined and incubated in the dark at 4°C for sixteen hours.  133 µl of $1 \times 10^{-4}$ M N-ethyl maleimide in methanol was added to the reaction mixture and incubated for thirty minutes at room temperature. The resulting mixture was dialyzed against two changes of low phosphate buffer (0.001 M sodium phosphate, 0.1 M sodium chloride, pH 7.0) (4 liters each). For purification of the SAV-BPE, a 1 ml column of hydroxyapatite was equilibrated with low phosphate buffer. The mixture was loaded and washed with 5 mls of low phosphate buffer collecting 1 ml fractions. In a stepwise fashion the elution buffers were applied (all 0.1 M NaCl, pH 7.0 plus either 0.003 M, 0.005 M, 0.008 M, 0.01 M, 0.03 M, 0.05 M, 0.1 M, or 0.4 M phosphate). The buffers were added according to increased molarity and changed when the eluent was clear. The desired fractions appeared after the 0.05 M phosphate buffer elution.

## EXAMPLE 6

The particles, to which the first fragment of nucleic acid was attached ("first fragment particles"), were centrifuged, resuspended to 0.25% (w/v) first fragment particles in pre-hybridization buffer (50% (v/v) formamide, 5x Denhardt's solution (100x = 2% BSA Pentex Fraction V (w/v), 2% Ficoll 400 (w/v) and 2% polyvinyl pyrrolidone (w/v)), 5x SSPE (1x = 0.18 M NaCl, 0.01 M sodium phosphate pH 7.7 and 1 mM EDTA) and 100 µg/ml denatured salmon sperm DNA, pre-warmed to 42° C). Incubation was for 2 hours at 42°C. The first fragment particles were centrifuged and resuspended in 42°C elution buffer (99% (v/v) formamide and 10mM Tris pH 7.8) for one hour. The first fragment particles were centrifuged and then resuspended in hybridization buffer to 0.25% w/v (50% formaminde v/v, 0.3 M sodium chloride, 0.003 M sodium citrate, 0.00025 M Tris pH 7.4, 0.00025 M EDTA and 100 µg/ml denatured salmon sperm DNA, pre-warmed to 42°C) and divided into aliquots of 100 μl per 0.5 ml microfuge tubes.

A mixture of 10 μg calf thymus DNA and 15 μg of labeled second fragment of DNA per 150 μl Tris buffer pH 7.5 was heat denatured at 100°C. 15 μl of this solution was added to a 0.5 ml microfuge tube containing 100 ml of the 0.25% first fragment particles in hybridization buffer. Incubation for ten hours was carried out at 42°C for reannealing to occur. Following hybridization the particles were washed 6 times with 50% v/v formamide and 0.2x SET (20x SET = 3 M NaCl, 0.4 M Tris pH 7.8 and 0.02 M EDTA) at 37°C. The resulting sandwich three-component coated particles were finally resuspended in 100 μl of 0.1 M phosphate buffer pH 7.0, forming "calf thymus DNA hybridization mixture".

Two additional hybridization mixtures were used as negative controls. First, a mixture of 10 μg of placental DNA and 15 μg of labeled second fragment of DNA per 150 μl Tris buffer pH 7.5 was heat denatured at 100° C as above. Second, 15 μg of labeled second fragment of DNA only per 150 μl Tris buffer pH 7.5 was heat denatured at 100° C as above. 15 μl of these control mixtures were added respectively to microfuge tubes containing 100 μl of the 0.25% first fragment particles in buffer and incubated, washed and resuspended as described above, forming "placental DNA control hybridization mixture" and "null control hybridization mixture" respectively.

### EXAMPLE 7

25 μl of the calf thymus DNA hybridization mixture was added to each of three wells of an automated front face 96 well assay plate fluorometer (Screen Machine 96, commercially available from Pandex Laboratories, Inc.,

Mundelein, Illinois). This was repeated for 25 $\mu$l of the placental DNA control hybridization mixture and the null control hybridization mixture respectively. It was also repeated with 50 $\mu$l of the calf thymus DNA hybridization mixture. Streptavidin-B-Phycoerythrin (SAV-BPE) was diluted in a diluent consisting of 1% normal goat serum and 1% fetal calf serum at a concentration of 2.5 $\mu$g per ml. The following protocol was followed for each of the wells containing hybridization mixture: add 20 $\mu$l diluted SAV-BPE, incubate 15 minutes, separate 2 minutes, wash with 60 $\mu$l isoton, separate 2 minutes, wash with 60 $\mu$l isoton, separate 2 minutes, and read with excitation wavelength 545 nm, emission wavelength 577 nm, gain setting times 5 to determine fluorescence.

## TABLE 1

| Well # | 25 $\mu$l of null control: | 25 $\mu$l of placental DNA control: | 25 $\mu$l of calf thymus DNA hybridization: | 50 $\mu$l of calf thymus DNA hybridization: |
|---|---|---|---|---|
| 1 | 1,414* | 1,594 | 6,576 | 12,402 |
| 2 | 1,752 | 1,792 | 6,552 | 14,010 |
| 3 | 1,772 | 1,924 | 6,294 - | 15,354 |
| Ave | 1,646 | 1,740 | 6,474 | 13,922 |

* Arbitrary fluorescence units

## EXAMPLE 8

10 $\mu$g of the 500 base pair fragment of Calf thymus DNA, purified as before, was added to terminal transferase buffer (0.22 M potassium cacodylate (pH 7.4), 0.01 M $MgCl_2$, 0.5 mM Hg-dUTP, 0.01 M ethyl mercaptan - all stocks made in 0.05 M Tris-Cl (pH 7.4). To this mixture, 284 units of terminal transferase was added. Incubation was carried out at 37°C for 30 minutes. The resulting Hg-dUTP terminally labeled DNA was attached to the particles as described above.

EXAMPLE 9

The foregoing sandwich assays were repeated except that (1) the incubation of Example 6 was reduced in duration from 10 to 2 hours and (2) the fluorometer reading of 50 μl of the calf thymus DNA hybridization mixture was replaced by 25 μl of a second calf thymus DNA hybridization mixture which was prepared using half as much calf thymus DNA as the original preparation in Example 6 (i.e. 5 ug of calf thymus DNA and 15 μg of labeled second fragment of DNA were added to 150 μl Tris buffer and heat denatured).

TABLE 2

| Well # | 25 μl of null control: | 25 μl of placental DNA control: | 25 μl of calf thymus DNA hybrid-ization: | 25 μl .5X reduced calf thymus DNA hybrid-ization: |
|---|---|---|---|---|
| 1 | 1,052 | 972 | 9,932 | 4,352 |
| 2 | 1,542 | 950 | 8,978 | 4,798 |
| 3 | 1,350 | 1,092 | 9,488 | 4,450 |
| Ave | 1,315 | 1,005 | 9,466 | 4,533 |

0200113

## CLAIMS

1.    An improved method of nucleic acid hybridization assay comprising:

allowing solid carrier bound three-component hybrid molecules to form by complementary base pairing of the following: (1) single-stranded sample nucleic acid to be assayed contained in a liquid sample of measurable volume; (2) single-stranded first fragment of nucleic acid having a nucleotide sequence of at least 15 bases and which is attached to a solid carrier, said first fragment of nucleic acid being capable of hybridizing with said sample nucleic acid; and (3) single-stranded second fragment of nucleic acid having a nucleotide sequence of at least 15 bases and to which is attached a luminescent label or a luminescent label acceptor to which a luminescent label may be attached subsequent to allowing the three-component hybrid molecules to form, said second fragment of nucleic acid being capable of hybridizing with said sample nucleic acid but not capable of hybridizing with said first fragment of nucleic acid; and

separating (1) the solid carrier bound three-component hybrid molecules to which luminescent label or luminescent label acceptor is attached from (2) luminescent label or luminescent label acceptor not attached to said solid carrier bound three-component hybrid molecules;

wherein the improvement comprises:

allowing complementary base pairing between the sample nucleic acid and the first fragment of nucleic acid to occur while the solid carrier comprising a plurality of water insoluble particles of about 50 microns or less in diameter, to which the first fragment of nucleic acid is attached, is in a substantially suspended state; and

measuring the luminescence of luminescent label attached to the three-component hybrid molecules while the solid carrier, to which such molecules are attached, has been concentrated by microfiltration to a volume substantially less than the volume of the liquid sample.

2.    The method of claim 1 wherein the three-component hybrid molecules are allowed to form by

first, allowing hybridization of the first fragment of nucleic acid with the sample nucleic acid while the solid carrier, to which the first fragment of nucleic acid is attached, is in a substantially suspended state, forming two-component hybrid molecules; and

second, allowing three-component hybrid molecules to form by complementary base pairing of the second fragment of nucleic acid with the sample nucleic acid contained in the two-component hybrid molecules.

3.      The method of claim 1 wherein the three-component hybrid molecules are allowed to form by simultaneous complementary base pairing of the sample nucleic acid, the first fragment of nucleic acid, and the second fragment of nucleic acid, while the solid carrier, to which the first fragment of nucleic acid is attached, is in a substantially suspended state.

4.      An improved method of nucleic acid hybridization assay comprising

allowing two species of solid carrier bound two-component hybrid molecules to form by complementary base pairing of the following: (1) single-stranded sample nucleic acid to be assayed contained in a liquid sample of measurable volume; (2) single-stranded first fragment of nucleic acid having a nucleotide sequence of at least 15 bases and which is attached to a solid carrier, said first fragment of nucleic acid being capable of hybridizing with said sample nucleic acid; and (3) single-stranded second fragment of nucleic acid having a nucleotide sequence of at least 15 bases and to which is attached a luminescent label or a luminescent label acceptor to which a luminescent label may be attached subsequent to allowing the two species of solid carrier bound two-component hybrid molecules to form, said second fragment of nucleic acid being capable of hybridizing with said first fragment of nucleic acid but not capable of hybridizing with said sample nucleic acid; and

separating (1) the two species of solid carrier bound two-component hybrid molecules to which luminescent label or luminescent label acceptor is attached from (2) luminescent label or luminescent label acceptor not attached to said two species of solid carrier bound two-component hybrid molecules;

wherein the improvement comprises:

allowing complementary base pairing between (1) the first fragment of nucleic acid and (2) the sample nucleic acid or the second fragment of nucleic acid to occur while the solid carrier comprising a plurality of water insoluble particles of about 50 microns or less in diameter, to which the first fragment of nucleic acid is attached, is in a substantially suspended state; and

measuring the luminescence of the luminescent label attached to two-component hybrid molecules while the solid carrier, to which such molecules are attached, has been concentrated by microfiltration to a volume substantially less than the volume of the liquid sample.

5.      The method of claim 4 wherein the two species of solid carrier bound two-component hybrid molecules are allowed to form by

first, allowing hybridization of the sample nucleic acid with an excess of first fragment of nucleic acid while the solid carrier, to which the first fragment of nucleic acid is attached, is in a substantially suspended state; and

second, allowing hybridization of (1) first fragment of nucleic acid which was not hybridized by the sample nucleic acid, with (2) the second fragment of nucleic acid.

6. The method of claim 4 wherein the two species of solid carrier bound two-component hybrid molecules are allowed to form by

first, allowing hybridization of the first fragment of nucleic acid with the second fragment of nucleic acid while the solid carrier, to which the first fragment of nucleic acid is attached, is in a substantially suspended state; and

second, allowing displacement of such hybridized second fragment of nucleic acid by hybridization of the first fragment of nucleic acid with the sample nucleic acid.

7. The method of claim 4 wherein the two species of solid carrier bound two-component hybrid molecules are allowed to form by simultaneous complementary base pairing of (1) sample nucleic acid and (2) second fragment of nucleic acid in competition for first fragment of nucleic acid, while the solid carrier, to which the first fragment of nucleic acid is attached, is in a substantially suspended state.

8. The method of claim 4 wherein the two species of solid carrier bound two-component hybrid molecules are allowed to form by

first, allowing hybridization of sample nucleic acid with the first fragment of nucleic acid, while the solid carrier, to which the first fragment of nucleic acid is attached, is substantially suspended; and

second, allowing competition for such first fragment of nucleic acid by hybridization of second fragment of nucleic acid with such first fragment of nucleic acid.

9.     An improved method of nucleic acid hybridization assay comprising

allowing two species of two-component hybrid molecules, one species bound to solid carrier and the other species not bound, to form by complementary base pairing of the following:  (1) single-stranded sample nucleic acid to be assayed contained in a liquid sample of measurable volume; (2) single-stranded first fragment of nucleic acid having a nucleotide sequence of at least 15 bases and which is attached to a solid carrier, said first fragment of nucleic acid not being capable of hybridizing with said sample nucleic acid; and (3) single-stranded second fragment of nucleic acid having a nucleotide sequence of at least 15 bases and to which is attached a luminescent label or a luminescent label acceptor to which a luminescent label may be attached subsequent to allowing the two species of two-component hybrid molecules to form, said second fragment of nucleic acid being capable of hybridizing with said sample nucleic acid or said first fragment of nucleic acid; and

separating the species of solid carrier bound two-component hybrid molecules from the species of unbound two-component hybrid molecules;
wherein the improvement comprises:

allowing complementary base pairing between (1) the first fragment of nucleic acid and (2) the second fragment of nucleic acid to occur while the solid carrier comprising a plurality of water insoluble particles of about 50 microns or less in diameter, to which the first fragment of nucleic acid is attached, is in a substantially suspended state; and

measuring the luminescence of the luminescent label attached to solid carrier bound two-component hybrid molecules while the solid carrier, to which such molecules are attached, has been concentrated by microfiltration to a volume substantially less than the volume of the liquid sample.

10. The method of claim 9 wherein the two species of two-component hybrid molecules are allowed to form by simultaneous complementary base pairing of (1) sample nucleic acid and (2) first fragment of nucleic acid in competition for second fragment of nucleic acid, while the solid carrier, to which the first fragment of nucleic acid is attached, is in a substantially suspended state.

11. The method of any of the claims 1 to 10 wherein the luminescent label is a fluorescent label or phosphorescent label or atomic fluorescent label or chemiluminescent label or bioluminescent label.

12. The method of any of the claims 1 to 11 wherein the luminescent label acceptor is formed by reaction of biotin with the second fragment of nucleic acid and the luminescent label is an avidin-luminescent label conjugate.

13. The method of any of the claims 1 to 12 wherein the insoluble particles are polymeric particles, preferably latex particles.

14. The method of any of the claims 1 to 13 wherein the water insoluble particles have a diameter in the range of about 0.2 microns to about 50 microns.

15. The method of any of the claims 1 to 12 wherein the luminescent label has an optical luminescence which is measured by a device having an excitation beam with a cross-section size within the range of about 1 to 5 mm.

16. The method of any of the claims 1 to 15 wherein the water insoluble particles are substantially transparent to a beam exciting the label and to a resulting luminescence emission beam.

17. The method of any of the claims 1 to 16 wherein the water insoluble particles are substantially transparent to luminescence resulting from chemical or biological excitation of the luminescent label.

18. The method of any of the claims 1 to 17 wherein the luminescence is measured while the solid carrier, which has been concentrated by microfiltration, has a cross-section size within the range of about 1 to 5 mm.

19. The method of any of the claims 1 to 18 wherein the sample nucleic acid is single-stranded DNA or RNA, the first fragment of nucleic acid is a single-stranded fragment of DNA or RNA and the second fragment of nucleic acid is a single-stranded fragment of DNA or RNA.